# FASCICULE DE BREVET EUROPEEN

(11) **EP 2 129 455 B1**
(45) Date de publication et mention de la délivrance du brevet: **23.04.2014**
(21) Numéro de dépôt: 08762097.7
(22) Date de dépôt: 14.02.2008
(51) Int. Cl.: B01F 17/14, B01F 17/42, B01J 13/00, B01F 3/08

(54) **PROCÉDÉ DE PRÉPARATION DE NANO-ÉMULSIONS**
VERFAHREN ZUR HERSTELLUNG VON NANOEMULSIONEN
METHOD FOR PREPARING NANO-EMULSIONS

(30) Priorité: 14.02.2007 WO PCT/FR2007/000269
(43) Date de publication de la demande: 09.12.2009
(73) Titulaire: Commissariat à l'Énergie Atomique et aux Énergies Alternatives, 75015 Paris (FR)
(72) Inventeur: BIBETTE, Jérôme, F-75005 Paris (FR); GOUTAYER, Mathieu, F-35400 Saint Malo (FR); TEXIER-NOGUES, Isabelle, F-38000 Grenoble (FR)
(74) Mandataire: Jacobson, Claude
(86) Numéro de dépôt international: PCT/FR2008/050249
(87) Numéro de publication internationale: WO 2008/104717

(56) Documents cités:
- EP-A- 0 406 162
- EP-A- 1 010 416
- EP-A- 1 018 363
- WO-A-2006/087156
- US-A1- 2006 257 493
- ZEEVI A ET AL: "THE DESIGN AND CHARACTERIZATION OF A POSITIVELY CHARGED SUBMICRON EMULSION CONTAINING A SUNSCREEN AGENT" INTERNATIONAL JOURNAL OF PHARMACEUTICS, ELSEVIER BV, NL, vol. 108, no. 1, 1 janvier 1994 (1994-01-01), pages 57-68, XP008013777 ISSN: 0378-5173
- T G MASON ET AL.: "Nanoemulsions: formation, structure and physical properties" J. PHYS.: CONDENS MATTER, vol. 18, 29 septembre 2006 (2006-09-29), pages R635-R665, XP002502173

## Description

La présente invention est relative à un procédé de préparation de nano-émulsions.

Les nano-émulsions, appelées quelquefois mini-émulsions, émulsions ultrafines ou encore émulsions submicroniques, sont des émulsions dans lesquelles la phase dispersée présente un diamètre moyen généralement compris entre 10 et 200 nm.

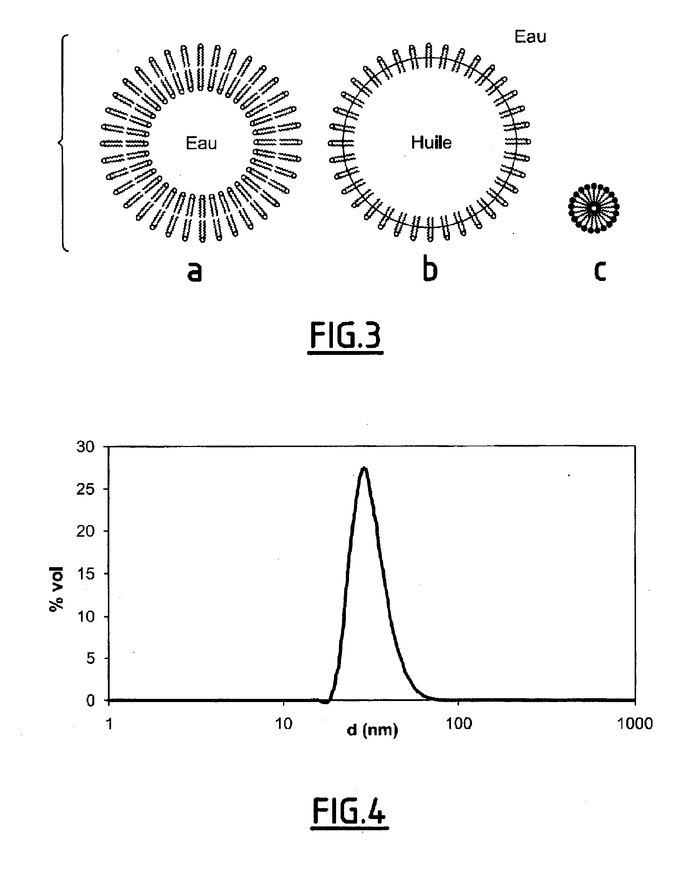

Pour rappel, une émulsion est un mélange de deux substances liquides non miscibles constitué d'une phase continue et d'une phase dispersée. Une substance est dispersée dans la seconde substance (phase continue) sous forme de petites gouttelettes (phase dispersée). Le mélange reste stable grâce à l'action de molécules amphiphiles, appelées émulsifiants ou tensioactifs, qui se placent à l'interface entre les deux phases. Les émulsions sont des édifices supramoléculaires métastables. Ces structures sont illustrées à la figure 3B et se différencient des polymersomes et des micelles.

Les polymersomes (famille comprenant les liposomes) sont des vésicules présentant un diamètre moyen variant de quelques dizaines à quelques milliers de nanomètres. Ces vésicules sont composées d'une ou de plusieurs bicouches de tensioactifs qui permettent de séparer le milieu intravésiculaire du milieu extérieur, les deux milieux étant de même nature, le plus souvent aqueux (Fig. 3A).

Les micelles consistent en des agrégats de tensioactifs autoassemblés, de quelques nanomètres de diamètre. Les tensioactifs s'organisent de manière à orienter leur partie hydrophile vers l'extérieur (le solvant) et leurs chaînes hydrophobes vers le coeur de la micelle (voir Fig. 3C).

Plusieurs procédés de préparation de nano-émulsions de type huile-dans-eau sont connus.

On a ainsi proposé un procédé dans lequel les tensioactifs sont solubilisés dans la phase continue, c'est-à-dire l'eau (Jafari, S. M. ; He, Y.; Bhandari, B., Production of sub-micron emulsions by ultrasound and microfluidization techniques. Journal of Food Engineering 2007, 82, (4), 478-488 et Jafari, S. M.; He, Y. H.; Bhandari, B., Nano-emulsion production by sonication and microfluidization - A comparison. International Journal of Food Properties 2006, 9, (3), 475-485 et Mason, T. G.; Wilking, J. N.; Meleson, K.; Chang, C. B.; Graves, S. M., Nanoemulslons: formation, structure, and physical properties. Journal of Physics: Condensed Matter 2006, 18, (4), R635-R666).

EP 1 018 363 et EP 1 010 16 décrivent le procédé de préparation d'une nanoémulsion huile dans l'eau pouvant contenir un lipide amphiphiles, notamment des phospholipides comprenant une étape de mélange d'une phase aqueuse et d'une phase huileuse sous agitation vive suivi d'une homogénéisation haute pression avec cisaillement. La nanoémulsion de EP 1 018 363 contient un tensioactif choisi parmi les copolymères blocs d'oxyde d'éthylène et d'oxyde de propylène et celle de EP 1 010 416 contient un tensioactif solide choisi parmi les esters gras de glycérol.

EP 0 406 162 décrit un procédé de préparation d'un nanoémulsion comprenant l'étape de mélange d'une phase aqueuse, d'une huile et d'un tensioactif amphotère dans un homogénéiseur haute pression.

WO 2006/087156 décrit un procédé de préparation de nanoémulsions comprenant des lipoaminoacides et des glycérides d'acides gras comme tensioactif comprenant les étapes de :
- préchauffage de la phase huileuse comprenant le lipoaminoacide et lesdits acides gras,
- mélange avec la phase aqueuse par application d'énergie mécanique dans des appareils émulsifiants.

Zeevi et al., International Journal of Pharmaceutics, 108(1), 57-68, 1994 décrit des émulsions submicroniques obtenues par :
- préparation d'une phase aqueuse comprenant éventuellement du poloxamer,
- préparation d'une phase huileuse comprenant de l'huile MCT, du α-tocophérol, des phospholipides,
- combinaison des deux phases chauffées suivie d'un mélange dans un mixeur à fort cisaillement.

US 2006/0257493 décrit un procédé de préparation de nanoémulsions huile dans eau comprenant les étapes de :
- préparer une phase aqueuse comprenant un lipide amphiphile et la chauffer à 70°C,
- préparer une phase huileuse et la chauffer à 70°C,
- mélanger les deux phases,
- homogénéiser.

Cependant, de nombreux tensioactifs, notamment les phospholipides, sont difficilement solubles dans la phase aqueuse et dans la phase huileuse. II est donc difficile d'incorporer dans l'une des phases la quantité de tensioactif nécessaire pour obtenir des nano-émulsions avec une phase dispersée de faible diamètre.

Afin de pouvoir solubiliser une quantité plus importante de tensioactif, il a été proposé de solubiliser les tensioactifs dans un solvant, lequel est ensuite évaporé de manière à former un film de tensioactifs. L'eau ou la solution aqueuse est ensuite ajoutée et le mélange est soniqué afin de disperser le film de tensioactifs. Dans la solution de liposomes obtenue est ensuite dispersée l'huile. Le mélange diphasique obtenu est à noweau soniqué ou traité au moyen d'un microfluidiseur pour donner la nano-émulsion (Vyas, T. K.; Shahiwala, A.; Amiji, M. M., Improved oral bioavailability and brain transport of Saquinavir upon administration in nouvel nanoemulsion formulations. International Journal of Pharmaceutics 2008, 347 (1-2), 93-101) et US 200510079131. Ce procédé est toutefois long et requiert plusieurs étapes de sonication. De plus, la présence de liposomes résiduels dans la nano-émulsion ne peut pas être exclue.

La présente invention a comme but principal de proposer un procédé de préparation de nano-émulsions permettant de surmonter l'un ou plusieurs des inconvénients cités ci-dessus.

En particulier, l'invention a comme but de proposer un procédé simple permettant l'accès à des nano-émulsions ayant une phase dispersée de faible diamètre.

L'invention a également comme but de proposer un procédé de préparation de nano-émulsions permettant de contrôler la taille de la phase dispersée et/ou la nature physico-chimique de l'interface.

Le but est atteint par un procédé de préparation d'une nano-émulsion dans lequel au moins un tensioactif peu soluble, notamment un phospholipide, est solubilisé dans la phase huileuse grâce à la présence d'un lipide solubilisant.

La présente invention a donc pour objet un procédé de préparation de nano-émulsions comprenant au moins une phase continue aqueuse et au moins une phase dispersée huileuse, comportant les étapes consistant à :
(i) préparer la phase huileuse comprenant au moins un lipide amphiphile et au moins un lipide solubilisant ;
(ii) disperser la phase huileuse dans une phase aqueuse sous l'action d'un cisaillement suffisant pour former une nano-émulsion ; et
(iii) récupérer la nano-émulsion ainsi formée
dans lequel le lipide solubilisant consiste en un mélange de glycérides d'acides gras saturés comportant 0 % à 20 % en poids d'acides gras en C8, 0 % à 20 % en poids d'acides gras en C10, 10 % à 70 % en poids d'acides gras en C12, 5% à 30% en poids d'acides gras en C14, 5% à 30% en poids d'acides gras en C16 et 5% à 30% en poids en C18.

Le procédé selon la présente invention présente en particulier les avantages suivants :
- il ne comporte qu'une seule étape de sonication ;
- il permet la dissolution de quantités importantes de tensioactifs et de ce fait la préparation de nano-émulsions dont la phase dispersée présente un faible diamètre ;
- il est rapide car la phase aqueuse est peu chargée en tensioactif et donc moins visqueuse et peut être émulsionnée rapidement; et
- il évite la formation de liposomes et permet ainsi l'accès à une nano-émulsion homogène.

Au sens de la présente invention :
- le terme « gouttelette » englobe à la fois les gouttelettes d'huile liquide proprement dites ainsi que les particules solides issues d'émulsions de type huile-dans-eau dans lesquelles l'huile utilisée est une huile cristallisable. Dans ce dernier cas, on parle souvent aussi d'émulsion solide.
- le terme « lipide » désigne de petites molécules principalement constituées de carbone, d'hydrogène et d'oxygène et présentent une densité inférieure à celle de l'eau. Les lipides peuvent être à l'état solide, comme dans les cires, ou liquide, comme dans les huiles.
- le terme « amphiphile » désigne des espèces chimiques possédant un groupe hydrophile et un groupe hydrophobe. Les composés amphiphiles ont des propriétés tensioactives, c'est-à-dire modifient la tension superficielle entre deux surfaces.
- le terme « phospholipide » vise des lipides possédant un groupe phosphate, notamment les phosphoglycérides. Le plus souvent, les phospholipides comportent une extrémité hydrophile formée par le groupe phosphate éventuellement substitué et deux extrémités hydrophobes formées par des chaînes d'acides gras. Parmi les phospholipides, on citera en particulier la phosphatidylcholine, la phosphatidyl éthanolamine, la phophatidyl inositol, la phosphatidyl sérine et la sphingomyéline.
- le terme « lécithine » désigne la phosphatidylcholine, c'est-à-dire un lipide formé à partir d'une choline, d'un phosphate, d'un glycérol et de deux acides gras. Il couvre de manière plus large les phospholipides extraits du vivant, d'origine végétale ou animale, dans la mesure où ils sont majoritairement constitués de phosphatidylcholine. Ces lécithines constituent généralement des mélanges de lécithines portant différents acides gras.
- le terme « nano-émulsion » désigne au sens de l'invention une émulsion dans laquelle la phase dispersée présente un diamètre moyen compris entre 10 et 200 nm, de préférence entre 10 et 150 nm et tout particulièrement entre 10 et 80 nm.

### [Phase huileuse]

La nano-émulsion préparée selon le procédé de l'invention comporte une phase huileuse et une phase aqueuse.

La phase huileuse comporte au moins un lipide amphiphile et au moins un lipide solubilisant.

Afin de former une nano-émulsion stable, il est généralement nécessaire d'inclure dans la composition au moins un lipide amphiphile à titre de tensioactif.

La nature amphiphile du ou des tensioactifs assure la stabilisation des gouttelettes d'huile au sein de la phase continue aqueuse. Le plus souvent, on utilise au moins deux tensioactifs pour préparer une nano-émulsion. Le tensioactif peut par ailleurs présenter d'autres effets dans l'application envisagée de la nano-émulsion.

Selon l'invention, la phase huileuse comporte au moins un lipide amphiphile.

Ces lipides amphiphiles peuvent être d'origine naturelle ou synthétique. De préférence, ils sont choisis parmi les phospholipides ; le cholestérol ; les lysolipides ; les sphingomyélines ; les tocophérols ; les glucolipides ; les stéarylamines et les cardiolipines.

La lécithine est le lipide amphiphile préféré.

Selon un mode de réalisation particulier, tout ou partie du lipide amphiphile peut posséder une fonction réactive, telle qu'un groupe maléimide, thiol, amine, ester, oxyamine ou aldéhyde. Cette variante permet le greffage de composés fonctionnels au niveau de l'interface. Le lipide amphiphile réactif est incorporé dans la couche formée à l'interface stabilisant la phase dispersée, où il est susceptible de se coupler avec un composé réactif présent dans la phase aqueuse.

Généralement, la phase huileuse comportera de 1 à 99% en poids, de préférence de 5 à 75% en poids et tout particulièrement de 20 à 60% en poids de lipide amphiphile.

Le lipide solubilisant est un lipide présentant une affinité avec le lipide amphiphile suffisant pour permettre sa solubilisation. Dans le cas où le lipide amphiphile est un phospholipide, il peut s'agir notamment de dérivés du glycérol, et en particulier de glycérides obtenues par estérification de glycérol avec des acides gras.

Le lipide solubilisant utilisé est avantageusement choisi en fonction du lipide amphiphile utilisé. Il présentera généralement une structure chimique proche, afin d'assurer la solubilisation recherchée. Il peut s'agir d'une huile ou d'une cire.

Les lipides solubilisants, en particulier pour les phospholipides sont les glycérides d'acides gras, notamment d'acides gras saturés, et en particulier d'acides gras saturés comportant 8 à 18 atomes de carbone, encore préféré 12 à 18 atomes de carbone.

Il s'agit de glycérides d'acides gras saturés comportant 0% à 20% en poids d'acides gras en C8, 0% à 20% en poids d'acides gras en C10, 10% à 70% en poids d'acides gras en C12, 5% à 30% en poids d'acides gras en C14, 5% à 30% en poids d'acides gras en C16 et 5% à 30% en poids d'acides gras en C18.

Particulièrement préférés sont les mélanges des glycérides semisynthétiques solides à température ambiante vendus sous la dénomination commerciale Suppocire^{®}NC par la société Gattefossé. Les Suppocire^{®} de type N sont obtenues par estérification directe d'acides gras et de glycérol. Il s'agit de glycérides hémi-synthétiques d'acides gras saturés de C8 à C18, donc la composition quali-quantitative est indiquée dans le tableau ci-dessous.

La quantité de lipide solubilisant peut varier largement en fonction de la nature et de la quantité de lipide amphiphile présent dans la phase huileuse.

Généralement, la phase huileuse comportera de 1 à 99% en poids, de préférence de 5 à 75% en poids et tout particulièrement de 10 à 50% en poids de lipide solubilisant.

**Tableau : Composition en acides gras du Suppocire NC^{®} de Gattefossé**

| Longueur de chaînes | [% en poids] |
|---|---|
| C8 | 0,1 à 0,9 |
| C10 | 0,1 à 0,9 |
| C12 | 25 à 50 |
| C14 | 10 à 24,9 |
| C16 | 10 à 24,9 |
| C18 | 10 à 24,9 |

De préférence, la phase huileuse comporte par ailleurs une ou plusieurs autres huiles.

Les huiles utilisées dans le cadre du procédé de l'invention présentent de préférence une balance hydrophile-lipophile (HLB) inférieure à 8 et encore plus préférentiellement comprise entre 3 et 6.

Avantageusement, les huiles sont utilisées sans modification chimique ou physique préalablement à la formation de l'émulsion.

Selon les applications envisagées, les huiles peuvent être choisies parmi les huiles biocompatibles, et en particulier parmi les huiles d'origine naturelle (végétale ou animale) ou synthétique.

Parmi de telles huiles, on peut notamment citer les huiles d'origine naturelle végétale parmi lesquelles figurent notamment les huiles de soja, de lin, de palme, d'arachide, d'olives, de pépin de raisins et de tournesol ; les huiles synthétiques parmi lesquelles figurent notamment les triglycérides, diglycérides et les monoglycérides. Ces huiles peuvent être de premières expression, raffinées ou inter-estérifiées.

Les huiles préférées sont l'huile de soja et l'huile de lin.

Généralement, la phase huileuse comportera de 1 à 70% en poids, de préférence de 2 à 50% en poids et tout particulièrement de 5 à 30% en poids d'huile.

Bien entendu, la phase huileuse peut contenir en outre d'autres additifs tels que des colorants, stabilisants, conservateurs, fluorophores ou principes actifs pharmacologiques en quantité appropriée.

Dans le cadre du procédé selon l'invention, on mélange d'abord les différents constituants huileux pour préparer un prémélange huileux pour la phase dispersée de l'émulsion. Le mélange peut éventuellement être facilité par mise en solution d'un des constituants ou du mélange complet dans un solvant organique approprié, suivie de l'évaporation du solvant, un prémélange huileux homogène pour la phase dispersée.

Par ailleurs, il est préféré de réaliser le prémélange à une température à laquelle l'ensemble des ingrédients sont liquides.

### [Phase aqueuse]

La phase aqueuse mise en oeuvre dans le procédé selon l'invention est de préférence constituée d'eau et/ou d'un tampon tel qu'un tampon phosphate comme par exemple du PBS ("Phosphate Buffer Saline") ou d'une solution saline, notamment de chlorure de sodium.

En outre, elle comporte éventuellement d'autres ingrédients, dont de préférence un co-tensioactif.

Les co-tensioactifs utilisables dans les émulsions conformes à la présente invention sont de préférence des tensioactifs hydrosolubles.

Les tensioactifs hydrosolubles comportent de préférence au moins une chaîne composée de motifs d'oxyde d'éthylène (PEO ou PEG) ou d'oxyde d'éthylène et d'oxyde de propylène. De préférence, le nombre de motifs dans la chaîne varie entre 2 et 500.

A titre d'exemple de co-tensioactifs, on peut en particulier citer les composés conjugués polyéthylèneglycol/phosphatidyl-éthanolamine (PEG-PE), les éthers d'acide gras et de polyéthylèneglycol tels que les produits vendus sous les dénominations commerciales Brij^{®} (par exemple Brij^{®} 35, 58, 78 ou 98) par la société ICI Americas Inc., les esters d'acide gras et de polyéthylèneglycol tels que les produits vendus sous les dénominations commerciales Myrj^{®} par la société ICI Americas Inc. (par exemple Myrj^{®} 45, 52, 53 ou 59) et les copolymères blocs d'oxyde d'éthylène et d'oxyde de propylène tels que les produits vendus sous les dénominations commerciales Pluronic^{®} par la société BASF AG (par exemple Pluronic^{®} F68, F127, L64, L61, 10R4, 17R2, 17R4, 25R2 ou 25R4 ) ou les produits vendus sous la dénomination commerciale Synperonic^{®} par la société Unichema Chemie BV (par exemple Synperonic^{®} PE/F68, PE/L61 ou PE/L64).

La phase aqueuse comporte de préférence de 0 à 50% en poids, de préférence de 1 à 30% en poids et tout particulièrement de 5 à 20% en poids de co-tensioactif hydrosoluble.

Selon un mode de réalisation préféré, la phase continue comporte également un agent épaississant tel qu'un glycérol, un saccharide, oligosaccharide ou polysaccharide, une gomme ou encore une protéine, de préférence du glycérol. En effet, l'utilisation d'une phase continue de viscosité plus élevée facilite l'émulsification et permet de ce fait de réduire le temps de sonication.

La phase aqueuse comporte avantageusement de 0 à 50% en poids, de préférence de 1 à 30% en poids et tout particulièrement de 5 à 20% en poids d'agent épaississant.

Bien entendu, la phase aqueuse peut contenir en outre d'autres additifs tels que des colorants, stabilisants et conservateurs en quantité appropriée.

Le prémélange aqueux pour la phase continue de l'émulsion peut être préparé par simple mélange des différents constituants avec le milieu aqueux choisi.

### [Emulsification]

Selon le procédé de l'invention, la nano-émulsion est préparée par dispersion de quantités appropriées de phase huileuse et de phase aqueuse sous l'effet d'un cisaillement.

La proportion de phase huileuse et de phase aqueuse est très variable. Cependant, le plus souvent, les nano-émulsions seront préparées avec 1 à 50%, de préférence 5 à 40% et tout particulièrement 10 à 30% en poids de phase huileuse et 50 à 99%, de préférence 60 à 95% et tout particulièrement 70 à 90 % en poids de phase aqueuse.

Avantageusement, la phase huileuse est ensuite dispersée dans la phase aqueuse à l'état liquide. Si l'une des phases se solidifie à température ambiante, il est préférable de réaliser le mélange avec les deux phases chauffées à une température supérieure ou égale à la température de fusion.

L'émulsification sous l'effet de cisaillement est de préférence réalisée à l'aide d'un sonificateur ou d'un microfluidiseur. De préférence, la phase aqueuse puis la phase huileuse sont introduites dans les proportions souhaitées dans un récipient cylindrique approprié puis le sonificateur est plongé dans le milieu et mis en marche pendant une durée suffisante pour obtenir une nano-émulsion, le plus souvent quelques minutes.

On obtient alors une nano-émulsion homogène dans laquelle le diamètre moyen des gouttelettes d'huile est supérieur à 10 nm et inférieur à 200 nm.

Avant conditionnement, l'émulsion peut être diluée et/ou stérilisée, par exemple par filtration. L'étape de filtration permet par ailleurs d'éliminer les éventuels agrégats qui pourraient s'être formés au cours de la préparation de l'émulsion.

L'invention sera expliquée plus en détail au moyen des exemples donnés à titre illustratif et des figures annexées, lesquelles montrent :

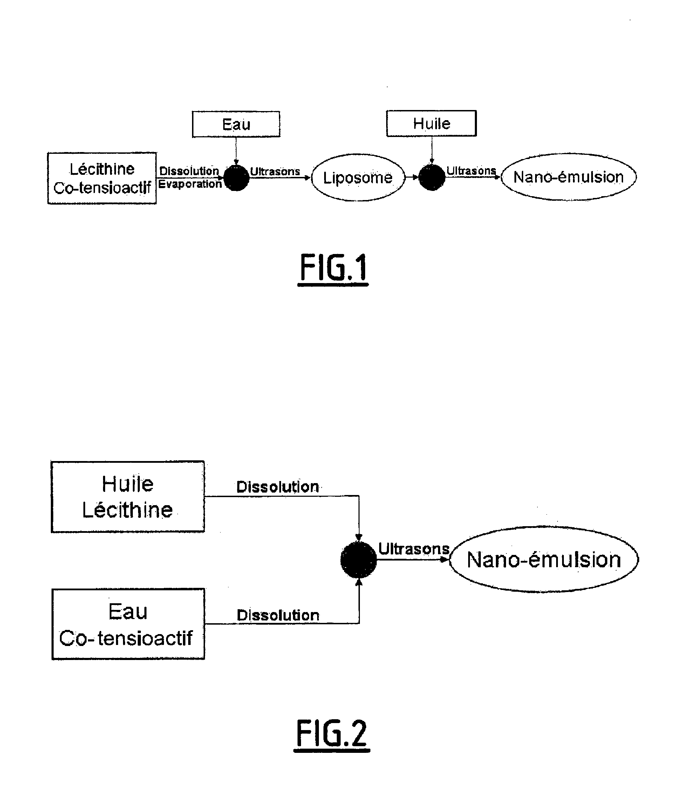

Fig. 1 : une représentation schématique de la production de nano-émulsions par ultrasons selon l'état de l'art ;
Fig. 2 : une représentation comparative de la production de nano-émulsions par ultrasons selon un mode de réalisation préféré de l'invention;
Fig. 3 : une représentation schématique de la structure d'une nano-émulsion (b) par rapport aux systèmes micellaires (c) et aux polymersomes (a), les structures ayant toutes de l'eau pour phase continue ;
Fig. 4 : la distribution en taille pondérée en volume d'une nano-émulsion préparée selon l'exemple 1, le pourcentage en volume étant indiqué fonction de la taille en nm ;
Fig. 5 : le diamètre moyen de la phase dispersée des nano-émulsions selon l'exemple 1 (■) et l'exemple 2 en fonction de la quantité totale de tensioactifs à ratio molaire tensioactif / co-tensioactif égal ;
Fig. 6 : le diamètre moyen de la phase dispersée des nano-émulsions selon l'exemple 1 (■) et l'exemple 3 en fonction de la quantité de tensioactifs et de co-tensioactifs; et
Fig. 7 : l'évolution dans le temps du diamètre moyen de nano-émulsions préparées selon l'exemple 1.

### EXEMPLES

### EXEMPLE 1_

### Formulation d'une nano-émulsion eau-dans-huile

Dans un récipient approprié, on a préparé un pré-mélange constitué de 0,125 g d'huile de soja (Sigma-Aldrich), de 0,375 g de glycérides semisynthétiques vendus sous la dénomination commerciale Suppocire^{®} NC (Gattefossé) et de 0,350 g de lécithine de soja (enrichie à 75 % de phosphatidylcholine) vendue par la société Lipoïd sous la dénomination commerciale Lipoïd^{®} S75.

Ces composés ont été dissous dans du chloroforme, la solution a ensuite été évaporée sous pression réduite et séchée à 50°C de manière à obtenir un prémélange se présentant sous la forme d'une huile visqueuse qui se solidifie en refroidissant. Le mélange obtenu est chauffé à 50-60°C de façon à le maintenir liquide pour l'émulsification.

La phase continue était préparée par mélange de 0,125 g de glycérol, de 0,55 g de stéarate de polyoxyéthylène à 50 moles d'oxyde d'éthylène vendu sous la dénomination commerciale Myrj^{®} 53 par la société ICI Americas Inc et de solution tampon phosphate, appelé PBS (acronyme anglais de Phosphate Buffer Solution) pour compléter à 4,150 g. Cette solution a été maintenue à chaud (50-60°C) avant émulsification.

La solution aqueuse a été ensuite ajoutée au mélange huile/lécithine. Puis la solution biphasique est mise en contact avec un sonificateur AV505^{®} équipé d'une sonde conique de 3 mm de diamètre (Sonics, Newtown) plongeant d'environ 1 cm dans la solution. La solution a été soniquée pendant 5 minutes avec le sonicateur réglé sur 30 % de la puissance maximale, avec une séquence de pulses suivante : 10 secondes de sonication / 30 secondes de repos. Durant la sonication, la solution a été maintenue à 40°C.

Le diamètre moyen de la phase dispersée de l'émulsion préparée est déterminé par diffusion dynamique de lumière (Zetasizer nano ZS, Malvern Instrument)

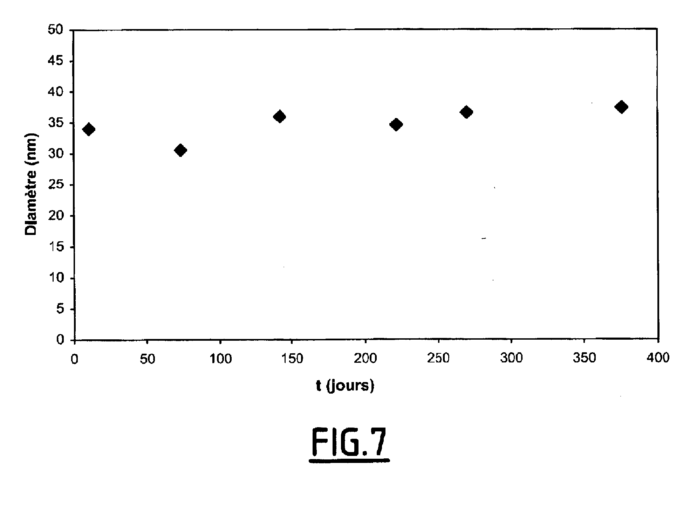

La phase dispersée des nano-émulsions ainsi obtenues présente un diamètre moyen de 35 nm (Fig. 4). Le tableau 1 résume la composition de la formulation de la nano-émulsion obtenue. La figure 7 illustre la stabilité dans le temps de nano-émulsions préparées selon l'exemple 1.

**Tableau 1 : formulation de la nano-émulsion de l'exemple 1**

| Exemple 1 | | Masse [mg] | % en poids | [mmol] |
|---|---|---|---|---|
| Phase dispersée | Huile de soja | 125 | 2,5 | |
| | Suppocire | 375 | 7,5 | |
| Tensioactifs | Lécithine | 350 | 7 | 0,467 |
| | Myrj 53 | 550 | 11 | 0,233 |
| Phase aqueuse | Glycérol | 125 | 2,5 | |
| | Solution tampon | 3475 | 69,5 | |
| Total | | 5000 | 100 | |

### EXEMPLE 2A à 2K

### Effet de la Quantité totale de tensioactif et co-tensioactif

Afin d'étudier l'effet des tensioactifs sur la formulation de l'exemple 1, celui-ci a été reproduit en faisant varier la quantité de lécithine et de Myrj 53, mais de manière à conserver le ratio molaire entre eux [lécithine / (lécithine + Myrj^{®} 53] constant à une valeur de 0,67. La quantité et la composition de la phase dispersée sont inchangées par rapport à la formulation de l'exemple 1. La quantité de solution tampon a été modifiée en conséquence pour obtenir 5 g de solution.

Dans les exemples 2A à 2B, où la quantité de co-tensioactif est importante, le glycérol est remplacé par de la solution tampon car la solution est suffisamment visqueuse. Le tableau 2 résume les concentrations utilisées pour les différentes formulations, préparées comme indiqué à l'exemple 1.

**Tableau 2 : formulation des émulsions des exemples 2A à 2K**

| Exemple | Tensioactif (lécithine) | | Co-tensioactif (Myrj^{®}53) | | Quantité totale de surfactant | Diamètre moyen |
|---|---|---|---|---|---|---|
| | Masse [mg] | % en poids | Masse [mg] | % en poids | [mmol] | [nm] |
| 1 | 350 | 7 | 550 | 11 | 0,7 | 36,6 |
| 2A | 420 | 8,4 | 660 | 13,2 | 0,84 | 35,1 |
| 2B | 385 | 7,7 | 605 | 12,1 | 0,77 | 34,2 |
| 2C | 315 | 6,3 | 495 | 9,9 | 0,63 | 37,4 |
| 2D | 280 | 5,6 | 440 | 8,8 | 0,56 | 40,3 |
| 2E | 245 | 4,9 | 385 | 7,7 | 0,49 | 45,7 |
| 2F | 210 | 4,2 | 330 | 6,6 | 0,42 | 72,7 |
| 2G | 175 | 3,5 | 275 | 5,5 | 0,35 | 92,9 |
| 2H | 140 | 2,8 | 220 | 4,4 | 0,28 | 127 |
| 21 | 105 | 2,1 | 165 | 3,3 | 0,21 | 170 |
| 2J | 70 | 1,4 | 110 | 2,2 | 0,14 | 177 |
| 2K | 35 | 0,7 | 55 | 1,1 | 0,07 | 272 |

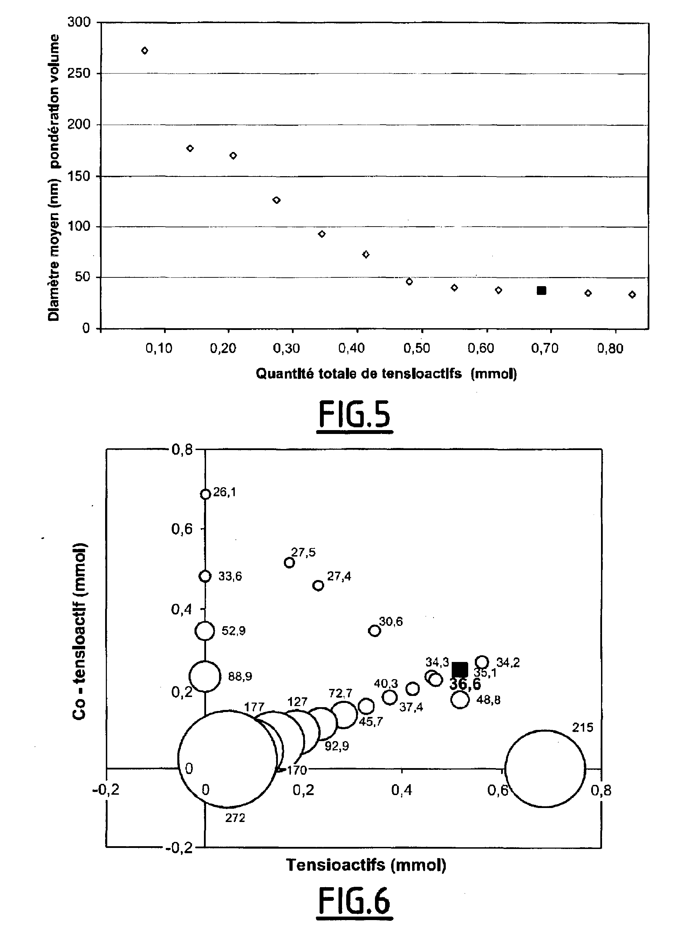

On constate que le diamètre moyen de la phase dispersée des nano-émulsions obtenues diminue avec lorsque la quantité totale de tensioactifs augmente (voir Fig. 5). La formulation de l'exemple 1 est représentée dans la figure 5 par un carré.

Il est donc possible de contrôler la taille de la phase dispersée de la nano-émulsion en ajustant la quantité de tensioactifs. On peut cependant distinguer deux régimes différents : en dessous de 0,5 mmol de tensioactifs, le diamètre moyen varie rapidement avec la quantité de tensioactifs, alors qu'au-delà de cette concentration, le diamètre moyen évolue peu. Les formulations comportant plus de 0,5 mmol de tensioactifs paraissent donc plus robustes étant donné qu'une faible variation de la quantité de tensioactif n'aura que peu d'effets sur la taille finale de la phase dispersée des nano-émulsions.

### EXEMPLES 3A à 3F

### Effet du ratio molaire tensioactif / co-tensioactif

L'exemple 1 a été ensuite reproduit mais en faisant varier le ratio tensioactifs / co-tensioactifs, en maintenant toutefois constant la quantité molaire totale de tensioactifs.

Dans les exemples 3A à 3F, où la quantité de co-tensioactif est importante, le glycérol est remplacé par de la solution tampon car la solution est suffisamment visqueuse. Le tableau 3 résume les concentrations utilisées pour les différentes formulations, préparées comme indiqué à l'exemple 1.

| Exemple | Tensioactif (lécithines) | | Co-tensioactif (Myrj^{®}53) | | Diamètre moyen |
|---|---|---|---|---|---|
| | Masse [mg] | % en poids | Masse [mg] | % en poids | [nm] |
| 1 | 350 | 7,0 | 550 | 11 | 34,3 |
| 3A | 516 | 10,3 | 0 | 0 | 215 |
| 3B | 387 | 7,7 | 428 | 9 | 48,8 |
| 3C | 258 | 5,2 | 856 | 17 | 30,6 |
| 3D | 172 | 3,4 | 1142 | 23 | 27,4 |
| 3E | 129 | 2,6 | 1284 | 26 | 27,5 |
| 3F | 0 | 0,0 | 1712 | 34 | 26,1 |

La taille de la phase dispersée des émulsions obtenues est indiquée en fonction de la concentration de lécithine et de Myrj^{®} 53, la formulation nominale étant représentée par un carré. Les données sont illustrées dans le diagramme de la figure 6.

On constate que la taille des gouttelettes diminue avec la quantité de tensioactif, mais aussi avec la quantité de co-tensioactif. On peut noter par ailleurs que l'effet du co-tensioactif paraît nettement plus important à cet égard.

Le ratio tensioactif / co-tensioactif permet donc également de contrôler le diamètre de la phase dispersée en affectant en même temps la composition de l'interface de l'émulsion.

### EXEMPLE 4

### Effet de la longueur des chaînes hydrophiles du co-tensioactif

Afin d'étudier l'effet de la longueur des chaînes hydrophiles du co-tensioactif, la formulation de l'exemple 1 a été reproduite mais en remplaçant le Myrij^{®} 53 par des co-tensioactifs portant des chaînes polyoxyéthylène de longueur différente.

Les co-tensioactifs suivants ont été testés : le Tween^{®} 80, le Myrj^{®} 45, le Myrj^{®} 49, et le Myrj^{®} 59. Le tableau 4 ci-dessous résume les caractéristiques de ces co-tensioactifs.

Le co-tensioactif dans la formulation de l'exemple 1 a été remplacé par la même quantité en mole, soit 0,233 mmol.

**Tableau 4 : Caractéristiques des co-tensioactifs polyéthoxylés**

| Co-tensioactif polyéthoxylé | Nombre d'unités polyoxyéthylène (POE) | Poids moléculaire (g/mol) |
|---|---|---|
| Tween^{®} 80 | 20 | 1310 |
| Myrj^{®} 45 | 8 | 636 |
| Myrj^{®} 49 | 20 | 1164 |
| Myrj^{®} 53 | 50 | 2484 |
| Myrj^{®} 59 | 100 | 4684 |

### EXEMPLE 5

### Effet de la longueur des chaînes hydrophiles du co-tensioactif dans une formulation comportant tensioactif et co-tensioactif en quantité équimolaire

L'étude de l'effet de la longueur des chaînes hydrophiles des co-tensioactifs a été approfondie par une seconde série d'expériences réalisée comme à l'exemple 4, mais sur la base d'une formulation dans laquelle le tensioactif et le co-tensioactif sont présents en quantité équimolaire (0,344 mmol).

Les résultats en terme de diamètre moyen de la phase dispersée sont résumés pour les exemples 4 et 5 dans le tableau 5 ci-dessous.

**Tableau 5 : Diamètre moyen de la phase dispersée des émulsions selon les exemples 4 et 5**

| Co-tensioactif | Exemple 4 [nm] | Exemple 5 [nm] |
|---|---|---|
| Tween^{®} 80 | 65,5 | 28,2 |
| Myrj^{®} 45 | 182 | - |
| Myrj^{®} 49 | 132 | 31,4 |
| Myrj^{®} 53 | 34,7 | 29,5 |
| Myrj^{®} 59 | 15,3 | 37,7 |

On constate que la longueur des chaînes hydrophiles dans le co-tensioactif a une influence certaine sur le diamètre moyen de la phase dispersée de l'émulsion obtenue pour les formulations de l'exemple 4. En effet, le diamètre moyen de la phase dispersée de l'émulsion diminue nettement lorsque la longueur de chaînes augmente.

Il est noté cependant que la nano-émulsion de l'exemple 4 avec un co-tensioactif présentant des chaînes très longues (Myrj^{®} 59) se révèle être instable. Le très faible diamètre de la phase dispersée (15,3 nm) laisse par ailleurs supposer la présence d'une solution micellaire plutôt qu'une nano-émulsion.

En revanche, la formulation équimolaire en tensioactifs de l'exemple 5 aboutit à des nano-émulsions dans lesquelles le diamètre de la phase dispersée reste proche de 30 nm. On peut donc, dans ce cas, changer la longueur des chaînes hydrophiles sans affecter de manière importante le diamètre des particules.

### EXEMPLE 6A à 6G

### Effet de la composition de la phase dispersée

Enfin, l'impact de la composition de la phase huileuse sur le diamètre moyen de la phase dispersée a été étudié en variant la nature et la proportion en huile et lipide solubilisant.

Dans la formulation selon l'exemple 1, la phase dispersée est composée d'un mélange de 75 % en poids de lipide solubilisant (Suppocire^{®} NC) et 25 % en poids d'huile de soja. Le lipide solubilisant est riche en acides gras de chaîne moyenne saturés, alors que l'huile de soja est riche en acides insaturés de type w6 (C18 :2) et l'huile de lin est riche en acides insaturés de type w3 (C18 :3).

On a donc préparé des nano-émulsions dont la phase dispersée est composée de lipide solubilisant pur, d'un mélange de lipide solubilisant et d'huile de soja ou d'huile de lin. La composition en lipide solubilisant et huile respective des nano-émulsions est indiquée en pourcentage en poids dans le tableau 8 ci-dessous.

Pour le reste, la formulation selon l'exemple 5 est conservée, cette formulation a comme avantage de réduire la quantité de lécithine par rapport à la formulation de l'exemple 1, permettant ainsi de diminuer la quantité de lipide solubilisant tout en assurant une bonne solubilisation de la lécithine. La formulation des nano-émulsions hors phase huileuse est résumée dans le tableau 7 ci-dessous.

**Tableau 7 : Formulation des nano-émulsions selon l'exemple 6**

| | | Masse mg | % | [mmol] |
|---|---|---|---|---|
| Phase dispersée | Huile totale | 500 | 10 | |
| Tensioactifs | Lécithine | 258 | 5 | 0,344 |
| | Myrj^{®} 53 | 827,5 | 16,55 | 0,344 |
| Phase aqueuse | Glycérol | 125 | 2,5 | |
| | Solution tampon | 3289,5 | 65,95 | |

Le tableau 8 ci-dessous résume le diamètre moyen de la phase dispersée dans les nano-émulsions obtenues avec la composition indiquée.

**Tableau 8 : Evolution du diamètre moyen en fonction de la composition de la phase huile**

| Exemple | Suppocire^{®} NC % en poids | Huile de soja % en poids | Huile de lin % en poids | diamètre moyen (d, nm) |
|---|---|---|---|---|
| 6A | 75 | 25 | 0 | 29 |
| 6B | 50 | 50 | 0 | 30,4 |
| 6C | 25 | 75 | 0 | 30,9 |
| 6D | 100 | 0 | 0 | 29,2 |
| 6E | 75 | 0 | 25 | 29 |
| 6F | 50 | 0 | 50 | 28,3 |
| 6G | 25 | 0 | 75 | 30 |

Ces résultats montrent que la composition de la phase dispersée de l'émulsion peut être modifiée sans faire varier son diamètre, permettant ainsi de modifier les caractéristiques de solubilisation sans affecter le diamètre de la phase dispersée des nano-émulsions.

Le procédé décrit permet donc d'obtenir facilement et rapidement des nano-émulsions stables et homogènes. Le procédé permet par ailleurs de contrôler le diamètre moyen de la phase dispersée, l'interface et/ou la phase dispersée des nano-émulsions par le biais de la concentration et du type de tensioactifs utilisés comme décrit ci-dessus.

## Revendications

1. Procédé de préparation de nano-émulsions comprenant au moins une phase continue aqueuse et au moins une phase dispersée huileuse, comportant les étapes consistant à :
(i) préparer la phase huileuse comprenant au moins un lipide amphiphile et au moins un lipide solubilisant;
(ii) disperser la phase huileuse dans une phase aqueuse sous l'action d'un cisaillement suffisant pour former une nano-émulsion; et
(iii) récupérez la nano-émulsion ainsi formée,
dans lequel le lipide solubilisant consiste en un mélange de glycérides d'acides gras saturés comportant 0% à 20% en poids d'acides gras en C8, 0% à 20% en poids d'acides gras en C10, 10% à 70% en poids d'acides gras en C12, 5% à 30% en poids d'acides gras en C14, 5% à 30% en poids d'acides gras en C16 et 5% à 30% en poids d'acides gras en C18.

2. Procédé selon la revendication 1, dans lequel le lipide amphiphile est choisi parmi les phospholipides, le cholestérol, les lysolipides, les sphingomyélines, les tocophérols, les glucolipides, stéarylamines, les cardiolipines d'origine naturelle ou synthétique.

3. Procédé selon la revendication 1 ou 2, dans lequel le lipide amphiphile est un phospholipide.

4. Procédé selon l'une des revendications 1 à 3, dans lequel la phase huileuse comporte en outre au moins une huile.

5. Procédé selon la revendication 4, dans lequel l'huile présente une balance hydrophile-lipophile (HLB) comprise entre 3 et 6.

6. Procédé selon l'une quelconque des revendications 1 à 5, dans lequel la phase huileuse comporte au moins une huile choisie parmi l'huile de soja et l'huile de lin.

7. Procédé selon l'une des revendications 1 à 6, dans lequel la phase aqueuse comporte en outre un co-tensioactif.

8. Procédé selon la revendication 7, dans lequel le co-tensioactif comporte au moins une chaîne composée de motifs d'oxyde d'éthylène ou d'oxyde d'éthylène et d'oxyde de propylène.

9. Procédé selon la revendication 7, dans lequel le co-tensioactif est choisi parmi les composés conjugués polyéthylèneglycol/phosphatidyl-éthanolamine (PEG-PE), les éthers d'acide gras et de polyéthylèneglycol, les esters d'acide gras et de polyéthylèneglycol et les copolymères blocs d'oxyde d'éthylène et d'oxyde de propylène.

10. Procédé selon l'une des revendications 1 à 9, dans lequel la phase continue de l'émulsion comprend un tampon physiologiquement acceptable.

11. Procédé selon l'une des revendications 1 à 10, dans lequel l'action de cisaillement est exercée par sonication.

12. Procédé selon l'une des revendications 1 à 11, dans lequel la phase huileuse est préparée par mise en solution de tout ou partie des constituants dans un solvant approprié et évaporation subséquente du solvant.

## Patentansprüche

1. Verfahren zum Herstellen von Nanoemulsionen, umfassend mindestens eine kontinuierliche wässrige Phase und mindestens eine dispergierte Öl-Phase, die die Schritte aufweist; die darin bestehen:
i) Herstellen der Öl-Phase, die mindestens ein amphiphiles Lipid und mindestens ein solubilisierendes Lipid enthält;
ii) Dispergieren der Öl-Phase in einer wässrigen Phase unter der Wirkung einer zur Bildung einer Nanoemulsion ausreichenden Scherung; und
iii) Gewinnen der so gebildeten Nanoemulsion,
bei dem das solubilisierende Lipid aus einer Mischung von gesättigten Fettsäureglyceriden, die 0 Gew.-% bis 20 Ges.-% C8-Fettsäuren, 0 Gew.-% bis 20 Gew.-% C10-Fettsäuren, 10 Gew.-% bis 70 Gew.-% C12-Fettsäuren, 5 Gew.-% bis 30 Gew.-% C14-Fettsäuren, 5 Gew.-% bis 30 Gew.-% C16-Fettsäuren und 5 Gew.-% bis 30 Gew.-% C18-Fettsäuren umfasst, besteht.

2. Verfahren nach Anspruch 1, bei dem das amphiphile Lipid ausgewählt ist aus Phospholipiden, Cholesterol, Lysolipiden, Sphingomyelinen, Tocopherolen, Glucolipiden, Stearylaminen, Cardiolipinen natürlichen oder synthetischen Ursprungs.

3. Verfahren nach Anspruch 1 oder 2, bei dem das amphiphile Lipid ein Phospholipid ist.

4. Verfahren nach einem der Ansprüche 1 bis 3, bei dem die Öl-Phase außerdem mindestens ein Öl aufweist.

5. Verfahren nach Anspruch 4, bei dem das Öl ein Hydrophilie-Lipophile-Gleichgewicht (HLB) zwischen 3 und 6 aufweist.

6. Verfahren nach einem beliebigen der Ansprüche 1 bis 5, bei dem die Öl-Phase mindestens ein Öl aufweist, das aus Sojaöl und Leinöl ausgewählt ist.

7. Verfahren nach einem der Ansprüche 1 bis 6, bei dem die wässrige Phase außerdem ein Co-Tensid aufweist.

8. Verfahren nach Anspruch 7, bei dem das Co-Tensid mindestens eine Kette aufweist, die aus Ethylenoxid-, oder Ethylenoxid- und Propylenoxid-Einheiten gebildet ist.

9. Verfahren nach Anspruch 7, bei dem das Co-Tensid ausgewählt ist aus den konjugierten Verbindungen Polyethylenglycol/Phosphatidyl-Ethanolamin (PEG-PE), Ethern von Fettsäuren und Polyethylenglycol, Estern von Fettsäuren und Polyethylenglycol und Blockcopolymeren von Ethylenoxid und Propylenoxid.

10. Verfahren nach einem der Ansprüche 1 bis 9, bei dem die kontinuierliche Emulsionsphase einen physiologisch annehmbaren Puffer enthält.

11. Verfahren nach einem der Ansprüche 1 bis 10, bei dem der Schervorgang durch Ultraschall ausgeübt wird.

12. Verfahren nach einem der Ansprüche 1 bis 11, bei dem die Öl-Phase durch vollständiges oder teilweises Lösen der Bestandteile in einem Lösungsmittel und nachfolgendes Verdampfen des Lösungsmittels hergestellt wird.

## Claims

1. Method for preparing nano-emulsions comprising at least one aqueous continuous phase and at least one oily dispersed phase, comprising the steps of:
(i) preparing the oily phase comprising at least one amphiphilic lipid and at least one solubilising lipid;
(ii) dispersing the oily phase in an aqueous phase under the effect of sufficient shear force to form a nano-emulsion; and
(iii) recovering the nano-emulsion thus formed,
wherein the solubilising lipid consists of a mixture of saturated fatty acid glycerides comprising 0 % to 20 % by weight of C8 fatty acids, 0 % to 20 % by weight of C10 fatty acids, 10 % to 70 % by weight of C 12 fatty acids, 5 % to 30 % by weight of C 14 fatty acids, 5 % to 30 % by weight of C16 fatty acids and 5 % to 30 % by weight of C18 fatty acids.

2. Method according to claim 1, wherein the amphiphilic lipid is selected from phospholipids, cholesterol, lysolipids, sphingomyelins, tocopherols, glucolipids, stearylamines or cardiolipins of natural or synthetic origin.

3. Method according to either claim 1 or claim 2, wherein the amphiphilic lipid is a phospholipid.

4. Method according to any one of claims 1 to 3, wherein the oily phase further comprises at least one oil.

5. Method according to claim 4, wherein the oil has a hydrophilic-lipophilic balance (HLB) of between 3 and 6.

6. Method according to any one of claims 1 to 5, wherein the oily phase comprises at least one oil selected from soybean oil and linseed oil.

7. Method according to any one of claims 1 to 6, wherein the aqueous phase further comprises a cosurfactant.

8. Method according to claim 7, wherein the cosurfactant comprises at least one chain composed of ethylene oxide units or ethylene oxide and propylene oxide units.

9. Method according to claim 7, wherein the cosurfactant is selected from the conjugated compounds polyethyleneglycol/phosphatidylethanolamine (PEG-PE), fatty acid and polyethyleneglycol ethers, fatty acid and polyethyleneglycol esters, and block copolymers of ethylene oxide and propylene oxide.

10. Method according to any one of claims 1 to 9, wherein the continuous phase of the émulsion comprises a physiologically acceptable buffer.

11. Method according to any one of claims 1 to 10, wherein the shear force effect is produced by sonication.

12. Method according to any one of claims 1 to 11, wherein the oily phase is prepared by placing all or some of the constituents in solution in an appropriate solvent and subsequently evaporating the solvent.
